# EUROPEAN PATENT SPECIFICATION

(11) **EP 2 099 436 B1**
(45) Date of publication and mention of the grant of the patent: **25.01.2017**
(21) Application number: 07865991.9
(22) Date of filing: 21.12.2007
(51) Int. Cl.: A61K 9/00, A61K 9/70, A61M 37/00, A61K 9/127, A61K 38/21, A61K 31/337

(54) **MICRODEVICE AND METHOD FOR TRANSDERMAL DELIVERY AND SAMPLING OF ACTIVE SUBSTANCES**
MIKROVORRICHTUNG UND VERFAHREN ZUR TRANSDERMALEN ABGABE UND PROBENAHME AKTIVER SUBSTANZEN
MICRO-DISPOSITIF ET PROCÉDÉ D'ADMINISTRATION ET DE PRÉLÈVEMENT TRANSDERMIQUE DE SUBSTANCES ACTIVES

(30) Priority: 22.12.2006 US 876948 P
(43) Date of publication of application: 16.09.2009
(73) Proprietor: Xu, Bai, Slingerlands, NY 12159 (US)
(72) Inventor: Xu, Bai, Slingerlands, NY 12159 (US)
(74) Representative: Westphal, Mussgnug & Partner Patentanwälte mbB
(86) International application number: PCT/US2007/088667
(87) International publication number: WO 2008/080109

(56) References cited:
- WO-A1-2005/002453
- WO-A1-2006/016647
- WO-A2-2005/041871
- WO-A2-2005/044366
- WO-A2-2005/069758
- WO-A2-2006/003659
- US-A1- 2005 261 631
- US-A1- 2005 261 632

## Description

### FIELD OF THE INVENTION

The present subject matter relates to a microdevice for transdermal delivery of an active substance and a method of using the same for cosmetic purposes.

### BACKGROUND OF THE INVENTION

Major reasons for the success of transdermal delivery were the avoidance of first- pass metabolism and ease of use. This increases drug bioavailability in comparison to other delivery methods. Transdermal Drug Delivery Systems (DDS) can also deliver drugs at a steady rate to achieve a sustainable release, which is an additional advantage. However, transdermal drug delivery methods have their drawbacks. Most important is the fact that conventional transdermal system (TTS) technology is only suited for delivering relatively small drugs across the skin. It also suffers from slow onset, because of the outer skin barrier layer, stratum corneum, that limits the through skin drug transport.

New transdermal drug delivery methods are therefore required to drive future growth in transdermal product markets. Biological products would also profit greatly from new, non-invasive delivery technology to replace hypodermic needle injection that is the
current standard. The original players in the transdermal field failed to introduce such improvements, which were then introduced by a number of innovator companies.

Broadly speaking, two different new approaches for transdermal drug delivery are currently being pursued: (1) nanoporation/minimum abrasion using a physical device, and (2) nanocarriers using lipid-encapsulated formulations. Sonoporation, thermoporation or use of very fine and short needles belong to the former; ultradeformable carriers (such as Transfersome ®, Ethosomes ® or fluid liposomes) are examples for the latter. Any of these can deliver small or large molecules across the skin. Some examples of transdermal delivery are described in U.S. Patent Nos. 7,094,423; 7,049,140; 7,041,870; 7,037,499; 7,034,126; 7,033,598; 7,014,855; 6,991,805; 6,982,084; and 6,979,729. Mention of State of the Art US 2005/261632 A1, US 2005/26161 A1 and WO 2006/003659 A2 disclose transdermal delivery that is obtained by pushing a plurality of microneedles or scratching a plurality of microblades, and microknives on a preselected side of the skin, and applying a skin patch containing active agents over the preselceted skin side. Further mention of the use of microdevices for that purposes are found in WO 2005/069758, WO 2005/0041871, WO 2005/002453 and WO 2005/044366.

However, there is a continuing need for an improved, disposable transdermal delivery device for effective delivery of substances in a controlled manner.

### SUMMARY OF THE INVENTION

It is an objective of the present subject matter to combine both nanocarriers and nanoporation methods to create new transdermal drug delivery vehicles.

It is a further objective of this subject matter to disclose a mechanical applicator to facilitate the application of nanoporation devices.

It is a still further objective of this subject matter to disclose a wet device/drug combination method to deliver a drug transdermally. The device/drug combination includes, but is not limited to: (1) pre-treatment of the mammal using the device, then applying the drug to the mammal; (2) applying the drug to the mammal, then treating the mammal with the device; (3) temporarily anchoring the drug onto the device, then treating the mammal with the device/drug system. In some embodiments, the method of delivering a therapeutically effective amount
of at least one cosmetic agent to a mammal in need thereof presented herein comprises applying an applicator device to an area of skin on the mammal to cause the device to generate a plurality of nanoconduits in stratum corneum of the area of skin, thereby creating a prepared area of skin;
applying a wet formulation to the prepared area of skin, wherein the wet formulation comprises at least one agent; and
allowing an effective amount of at least one agent to enter the mammal via the nanoconduits in the stratum corneum;
wherein the applicator device comprises an element capable of generating a plurality of nanoconduits in the stratum corneum.

In some embodiments, the present subject matter provides an applicator of the microdevice described herein.

### BRIEF DESCRIPTION OF THE DRAWINGS

Figures IA and IB show two preparations of liposome nanoparticles containing docetaxel.
Figure 2 shows penetration (%) of docetaxel in elastic liposomes with or without microneedle.
Figure 3 shows fluorescence labeled docetaxel encapsulated within elastic liposome nanoparticles being successfully transported through skin.
Figure 4 shows delivery of interferon via different methods.
Figure 5 shows delivery of interferon with a dry formulation.
Figure 6 shows laser confocal scanning micrographs of diffusion of Rhodamine B through the skin.
Figure 7 shows the in vitro transdermal delivery of insulin via nanoconduits in skin barrier between reservoirs.

### DETAILED DESCRIPTION OF THE INVENTION

The present subject matter provides methods of making and using nanoconduits for transdermal delivery of active agents. The present subject matter provides high-aspect- ratio microstructures (HARMS) and methods of using the same to generate nanoconduits. The present subject matter also provides methods of using the device for transdermal delivery of drugs, vaccines, diagnostic agents and cosmetic substances and sampling of body fluids for treating, preventing, or ameliorating a medical condition of a mammal such as a human being. In some embodiments, the method comprises treating a topical site of a mammal using a device, and applying an effective amount of an agent to the topical site to allow the agent to penetrate into the body of the mammal. The device can include an array of microstructures. The microstructure can have an aspect ratio of about 5:1, 10:1, 15:1, 20:1 or higher.

The term "nanoconduits" as used herein refers to microscale or nanoscale transdermal conduits directly or indirectly lined with tissue displaced by an element or device that penetrates the tissue to create the conduits. Nanoconduits are pores, channels, and conduits that are generated to traverse the vertical thickness of the stratum corneum of the skin. Nanoconduits thereby facilitate the transport of materials, such as the agents of the present subject matter, from outside the skin into the viable epidermis layer that lies beneath the stratum corneum. The term nanoconduit is used interchangeably with the terms nanopore and nanochannel. Nanoconduits generated in the skin (a) have a depth about equal to or slightly greater than the thickness of the stratum corneum, (b) have an optional depth that penetrates into the viable epidermis layer, and (c) have an optional depth that does not penetrate to the blood vessels, lymphatics, or nerves in the dermis layer. The present nanoconduits allow the permeation into the skin and past the stratum corneum of an agent having a molecular weight higher than about 500 Daltons. The present nanoconduits also allow for enhanced permeation into the skin and past the stratum corneum of an agent having a molecular weight less than about 500 Daltons, as compared to skin without nanoconduits present.

"Microscale" or "nanoscale" transdermal conduits line the tissue displaced by said applied microdevice or element. Microscale refers to dimensions on the order of one to hundreds of micrometers in scale. Nanoscale refers to dimensions on the order ranging from tens of nanometers to tens of thousands of nanometers. In one embodiment herein, the tip of the microdevice (or width of the edge of the microblade or microknife) is ten thousand nanometers in width or smaller and the body of the microdevice is 10-20 micrometers in width or higher. The length of the microdevices are generally of a microscale, having a length ranging, for example, from a few microns to a hundred microns in length or longer. In one embodiment herein, the length of the microdevice corresponds to the depth that one would like to generate the nanoconduits into the skin. The microdevice may be in the form of needles, blades, or combinations thereof, having a nanoscale width tip and a microscale width body. In the present microstructures, the tip of the microneedle or the edge of the microblade and microknife needs to be sharp in order to lower the insertion force, while the body of the microdevice should be high enough to allow it to completely penetrate the stratum corneum.

The ratio of tip width to device length is referred to as the "aspect ratio". Aspect ratios of the preferred devices herein are usually about 10:1 or higher, with the size of the tip and edge smaller than 5 microns and the height of microdevices higher than 50
microns. High-aspect-ratio microstructures (HARMS) typically have an aspect ratio higher than about 5:1 and they may be useful for a variety of purposes. HARMS can thus be used to fabricate microdevices including microneedles, microblades, and microknives for drug delivery through skin or body fluids extraction out of skin. Another example of HARMS is nanochannels for microfiuidic manipulation and transport. HARMS is typically made by Micro-ElectroMechanical Systems (MEMS) and nanofabrication technology that involves a number of thin film deposition, photolithography, etching and electroplating, injection molding, hot embossing, self-assembly, as well as LIGA process.

In some embodiments, the present subject matter provides a system for topical or systemic delivery of an agent for a medical condition in a mammal (e.g., a patient). The system comprises: (1) a microdevice comprising an array of microstructures, (2) an applicator for applying the microdevice to an area of skin of a patient to generate a prepared area of skin comprising a plurality of nanoconduits in stratum corneum of the prepared area of skin, and (3) a delivery mechanism for causing the agent to be delivered to the mammal through the nanopores or nanochannels in the stratum corneum of the prepared area of skin, hi some embodiments, the microdevice can comprise nanoscale tips and a microscale body that can have an aspect ratio of about 5:1, 10:1, 15:1, 20:1 or higher.

Accordingly, one aspect of the present subject matter relates to a method of delivering a therapeutically effective amount of at least one cosmetic agent to a mammal in need thereof, comprising
applying an applicator device to an area of skin on the mammal to cause the device to generate a plurality of nanoconduits in stratum corneum of the area of skin, thereby creating a prepared area of skin;
applying a wet formulation to the prepared area of skin, wherein the wet
formulation comprises at least one agent; and
allowing an effective amount of at least one agent to enter the mammal via the nanoconduits in the stratum corneum;
wherein the applicator device comprises an element capable of generating a plurality of nanoconduits in the stratum corneum.

Also disclosed is a method of delivering an agent for a medical condition to a mammal. The method comprises: (1) applying an applicator to a microdevice to cause the microdevice to contact an area of skin to generate a prepared area of skin comprising a plurality of nanopores or nanochannels through the stratum corneum of the area of skin, (2) applying a composition comprising the agent to the prepared area of skin, and (3) causing an effective amount of the agent to deliver to the patient through the nanopores or nanochannels in the stratum corneum.

In some embodiments, the method of delivering an agent described herein includes: (1) applying a composition comprising the agent to an area of skin, (2) applying an applicator to a microdevice to cause the microdevice to contact the area of skin to generate a plurality of nanopores or nanochannels through the stratum corneum of the area of skin, and (3) causing an effective amount of the agent to deliver to the patient through the nanopores or nanochannels in the stratum corneum.

In some embodiments, the method of delivering an agent described herein includes: (1) applying an applicator to a microdevice to cause the microdevice to contact an area of skin of a mammal (e.g., patient) to generate a prepared area of skin comprising a plurality of nanopores or nanochannels through the stratum corneum of the area of skin, and (2) causing an effective amount of an agent to deliver to the patient through the nanopores or nanochannels in the stratum corneum. The microdevice can be coated with a composition comprising the agent. In some embodiments, the causing is achieved by
applying a wet formulation including elastic liposomes comprising liposome nanoparticles encapsulating the agent and causing the agent to transport through the stratum corneum into the mammal. In some embodiments, the wet formulation does not include elastic liposomes.

As used herein, the term "composition" sometimes can be used interchangeably with the term "formulation." The term "wet formulation" refers to any form of wet formulation with water content higher than 1% by weight, or which is otherwise in some sort of liquid or partially liquid form. Preferred embodiments of wet formulations contain more than 2% water by weight. In some embodiments, a wet formulation can be a skin patch, cream, ointment, gel, patch, elastic liposome, lotion, or combination thereof. Wet formulations may include aqueous and non-aqueous compositions. In some embodiments, the wet formulation can include elastic liposomes comprising liposome nanoparticles encapsulating an agent. In some embodiments, the wet formulation can include an agent, but not elastic liposomes.

As used herein, the term "agent" refers to any diagnostic, therapeutic, cosmetic, or preventive agent. The term "agent" is sometimes interchangeably referred to as "active agent," "bioactive agent," or "active substance." Bioactive agents refer to agents that have at least one known and specific bioactivity. Bioactive agents may also be diagnostic agents or therapeutic agent. Therapeutic agents are agents that provide a therapeutic or preventative effect. "Therapeutic effect" refers to the ability of an agent to provide a beneficial effect on a biological organism, such as, for example, to prevent or treat a disease or disorder. A therapeutically effective amount refers to an amount of agent to effect prevention or treatment of a disease or disorder.

### Skin Structure

Skin has a biological barrier called stratum corneum in its outer layer. This layer of about 10-25 microns thick prevents most molecules from penetrating through the skin. The layer below the stratum corneum is called viable epidermis. Epidermis is between 50 to 100 micron thick. The viable epidermis layer has no blood vessels and the molecules in this layer can be transported to and from the dermis, a layer under the viable epidermis, which is between 1 to 3 mm thick. There are blood vessels, lymphatics and nerves in dermis layer. To date, for example, a skin patch is only able to deliver drug molecules of less than 500 Da through the skin. In addition, those small molecules that can be delivered through the skin are typically limited to hydrophobic ones. Drug molecules of greater than 500 Da cannot be readily delivered through the skin.

The phrase "intraepidermal drug delivery" as used herein refers to a method of some embodiments of drug delivery to a site that is in between the dermis and the stratum corneum. By effectively breaking the stratum corneum and delivering the drug to the epidermal layer, the drug, large or small, can easily diffuse through the epidermal and dermal layers into the mammal's body.

### Requirement of Delivery of Drugs, Vaccines and Cosmetic Substances

Successful transdermal delivery of therapeutic drugs, vaccines and cosmetic substances needs a way to transport molecules, especially large molecules, through the skin barrier, i.e., the stratum corneum. The substance can be delivered into the skin in any form acceptable for pharmaceutical requirements, but a gel composition is preferred to achieve controlled release of the active ingredient(s).

The microdevice described herein can be used for effective transdermal delivery of an agent or a combination of agents. The microdevice can be a microdevice array comprises a plurality of microstructures formed of a metallic, semi-conductor, glass, ceramic, or polymeric material. In some embodiments, the microdevice can be a microneedle, microknife, or microblade. In some embodiments, the microdevice
comprises microstructures having a nanoscale tip or edge and a microscale body.

### Microdevices

The microdevices described herein can be any of microneedles, microblades, microknives, or combinations thereof. As used herein, the term "microneedle" means an elongated element having a ratio of length to largest cross-sectional dimension, of at least about 1:1 or higher. The microneedle may have a regular or irregular cross-sectional shape, such as, for example, circular, elliptical, geometric, or a combination thereof. The needle may optionally include one or more edges running part of or all of the length of the needle's central axis of elongation. The term "microneedle" may also refer to a means that is sufficiently sharp to puncture skin tissue, such as the stratum corneum, to thereby generate nanoconduits.

As used herein, the terms "microblade" and "microknife" both mean an elongated element that is substantially long and thin. For example, having a ratio of length to thickness, of at least about 2:1 or higher. Each microblade includes two surfaces that meet at a single long edge. The length of the edge may be oriented parallel to the skin surface, perpendicular to the skin surface, or at an angle to the skin surface. A microblade with an edge that is significantly parallel to the skin surface may generate a nanoconduit in the skin that is an elongated opening or channel along the surface of the skin that has a narrow width and a depth into the skin as described above for nanoconduits. A microblade with an edge that is significantly perpendicular to the skin surface may generate a nanoconduit in the skin that is an elongated opening in the skin that has a narrow width, or the nanoconduit may have more of a puncture shaped volume wherein the opening has narrow widths in all directions. The terms "microblade" and "microneedle" may also refer to a means having an elongated edge that is sufficiently sharp to puncture skin tissue, such as the stratum corneum, to thereby generate nanoconduits. A microdevice may comprise one
or more microneedles, microblades, or microknives.

A "microneedle array" as used herein refers to a localized arrangement of more than one microneedle, microblade, microknife, or combinations thereof on a surface. The localized arrangement may comprise regular or irregular patterns of the microneedles, microblades, or microknives. For example, microneedles and/or microblades may be arranged in rows, in random formation, or a combination thereof. The microneedle, microblade, microknife, or microneedle array may be present on one or more surfaces of an applicator device.

The microdevice can further include microchannels and microreservoirs. The microdevice or microneedle array may also optionally comprise hollows, voids, non- smooth textures, and/or cavities. One possible use of the hollows, voids, non-smooth textures, and/or cavities is as a site to localize, concentrate, or deliver the agent in embodiments where the agent is to be applied to the skin simultaneously with the generation of the nanoconduits. In some embodiments, the microneedles or microblades comprise hollow channels for delivering an agent via diffusion or active injection, for example from a reservoir. In other embodiments, the microneedles or microblades do not comprise hollow channels for delivering an agent via diffusion or active injection. Some examples of the microdevice are described in U.S. application Serial Nos. 10/908,584, filed on May 18, 2005 and 11/510,078, filed on August 25, 2006.

### Materials and Device Sterilization

The microdevices herein can be made of many different materials or their combinations, including metals, ceramics, silicon, glass, polymers, and combinations thereof. Examples of the materials are titanium, stainless steel, nickel, alloy of nickel-iron, silicon, silicon oxide, glass, polymethyl methacrylate (PMMA), polyaryletherketone,
nylon, PET, poly(lactic acid), poly(glycolic acid) (PGA), poly(lactic-co-glycolic acid) (PLGA), polycarbonate, and polystyrene. The devices should have enough mechanical strength to penetrate skin without breaking and/or buckling while ensuring delivery of the agent(s), or collection of biological fluids. They can be sterilizable using established protocols (see, for example, moist heat, ethylene oxide or radiation sterilization as stated by AN-SLAAMFISO 11134:1993, ANSI/AAMI/ISO 11135:1994 and ANSI/AAMI/ISO 11137:1994 ).

### Elastic liposome

An elastic liposome is an artificial vesicle designed to be like a cell vesicle, and used to deliver drugs or genetic material into a cell. Its bounding membrane is more flexible than that of a liposome, allowing it to deform and pass through openings in a barrier, such as the skin, whose diameters are much smaller than the average vesicle size. An elastic liposome is an at least bi-component, most often vesicular, aggregate. The main functional characteristic of the aggregate is the extreme flexibility and permeability of its bilayer-like membrane coating. Its basis is the interdependency of local membrane shape and composition, which makes the bilayer self-regulating and self-optimizing. The bilayer is thus capable of stress adaptation, via local and reversible bilayer component demixing. All this makes an elastic liposome into a tool suitable for non-invasive and targeted drug delivery, for example across intact skin. Another beneficial consequence of high bilayer flexibility is the increased elastic liposomes affinity to bind and retain water. Ultradeformable elastic liposome vesicles put in a dry environment therefore seek to find a water richer region. This forces elastic liposome vesicles applied on open skin to penetrate the skin barrier in a search for adequate hydration. The resulting vesicle migration is a consequence of continuous bilayer
adaptation and deformation, but must not compromise unacceptably either the vesicle integrity or the protective skin barrier properties in real-life applications.

A basic elastic liposome is composed of one natural amphiphat (such as phosphatidylcholine) that tends to self-aggregate into vesicles. The latter are then supplemented by at least one bilayer softener (e.g. a biocompatible surfactant). The vesicle-like elastic liposome thus normally possesses an aqueous core surrounded by a complex, very fluid and adaptable lipid bilayer. While its basic organization is broadly similar to a simple lipid vesicle (also called a liposome), an elastic liposome differs from the latter by its more flexible and permeable, "softened" bilayer membrane. An elastic liposome vesicle can consequently change shape readily and easily by adjusting the relative concentration of its two components in the bilayer to the local stress experienced by the complex bilayer. This can be observed indirectly by studying stress- or deformation-dependent vesicle bilayer elasticity or permeability. In a single experiment, the same goal can be achieved by determining the pressure dependency of elastic liposome suspension-flux through a nano-porous filter (with the pores considerably smaller than the average vesicle size). The rate of resulting transport must grow with driving force (head pressure) non-linearly (often sigmoidally) until maximum flow is reached. For an ideal elastic liposome, experiencing no friction in pores, the maximum flow is equivalent to the flux of the suspending liquid measured with a similar trans-filter pressure, and the minimum pressure required to attain good transport is a measure of bilayer flexibility. The observed functional dependency of suspension flux versus pressure can therefore be used to derive bilayer elasticity and flexibility, as well as permeability, based on theoretical description of the underlying enforced transport, viewed as an activated transport process.

### Liposome carrier

In some embodiments, the delivery formulations described herein include a carrier that comprises elastic liposomes. hi some embodiments, the liposomes can be nanoparticles containing lipid-encapsulated therapeutic agents. The liposomes or nanoparticles are complex, most often vesicular, aggregates. In some embodiments, the liposomes or nanoparticles can be optimized to attain a flexible and self-regulating membrane, which makes the vesicle very deformable. These liposomes or nanoparticles can be typically applied on the skin and can be engineered to achieve a high drug concentration at or near the site of application, diminishing local or systemic adverse side effects, and often increasing drug potency. The tern "high drug concentration" refers to a local concentration sufficient enough to achieve desired therapeutic effects without incurring significant side-effects.

Liposome nanoparticles can be formed by any known method in the art. Generally, the method for forming liposome nanoparticles can be by thin film dispersion, reverse-phase evaporation, alcohol infusion, or extrusion with or without pressure, which are known in the art (see, e.g., Planas ME.;Gonzalez M.E.;Rodriguez L.et al., "Noninvasive percutaneous induction of topical analgesia by a new type of drug carrier, and prolongation of local pain insensitivity by anesthetic liposomes", Anesth.Analg 1992. 75(4):615-621 ; Gregor Cevc, Gabiele Blume, Andreas S. et al., "The skin pathway for systemic treatment with patches and lipid based agent carries", J. Advanced Drug Delivery Reviews, 18:349 (1996); Gregor Cevc, et al., "Ultradeformable lipid vesicles can penetrate the skin and other semi-permeable barriers unfragmented. Evidence from double label CLSM experiments and direct size measurements", Bio-chimica et Biophysica Acta 1564:21-30 (2002); G. Cevc, et al., "Overcoming semipermeable barriers, such as the skin, with ultradeformable mixed lipid vesicles, Transfersomes(R) liposomes or mixed lipid micelles", Langmuir, 19:10753-10763 (2003); Gregor Cevc, "Lipid vesicles and other colloids as drug carriers on the skin", Advanced Drug Delivery Reviews 56:675-711 (2004) ).

Figures IA and IB show two preparations of liposome nanoparticles containing docetaxel.

### Method of Use

The device described herein can be used for transdermal delivery of an agent or a combination of agents to treat, prevent, or ameliorate a body condition in need of treatment. The method generally includes treating a skin site of delivery with a microdevice described herein, and delivering an agent to the body of a mammal (e.g., a user or patient).

An applicator and method is described for applying a microneedle/nanoporation device, including a plurality of microneedles with a gentle impact. The method is used to improve transport of an active agent through the skin barrier.

It is noteworthy that the prior art uses drug coated tip or hollow microneedles to deliver drug through the skin. In contrast, the present subject matter provides for a method that includes, e.g., pre-treating skin by a microneedle array to generate a pre-treated area of skin, and applying to the pre-treated area a wet formulation to allow a therapeutic agent (e.g., a drug) or a combination of therapeutic agents to transport through the skin. The wet formulation can be in the form of a lotion, cream, gel patch, ointment or skin patch.

In some embodiments, the agent can be included in the microdevice as a coating with or without a carrier. In these embodiments, the agent can be delivered with the microdevice being attached to the site of delivery until a desired quantity or duration of delivery is achieved.

In some embodiments, the agent can be separate from the microdevice. In these embodiments, the skin site chosen for delivery of the agent can be pre-treated with the microdevice. The agent can then be applied to the skin site of delivery to allow the agent to penetrate into the body of a user or patient.

The body condition can be a cosmetic condition.

Representative cosmetic conditions include, but are not limited to, skin aging, skin wrinkle, dark spot, skin discoloration, moisturizing, skin lightening, skin whitening, skin firming, skin lifting, acne, wart, infection, irritation, dry skin and oily skin. Various combinations of any of the conditions herein are further contemplated herein.

The present microdevices are designed as disposable or re-usable devices. In one embodiment, the microdevices are disposable. Depending on whether or not the microdevices have a coating of active substances on them, there are three categories of applications in the delivery of drugs, cosmetic substances and vaccines in a preferred embodiment.

For delivery of a cosmetic substance, in one embodiment, the microdevices can be used to perforate or scratch the stratum corneum. They are then removed immediately and a formulation of an active substance such as a lotion, cream, gel patch, ointment or skin patch with the active substance is applied to the microdevice treated area right away. The formulation will stay on the skin for a pre-defined period,
providing sustainable controlled release of an agent such as a drug, or a combination of agents. In a preferred embodiment, the formulation is a wet skin patch.

Another embodiment is to store the active agents, as defined below, in the substrate and rely on passive diffusion when the microdevice is in touch with skin.

In yet another embodiment, one can apply the drugs, in the forms of a gel, cream, ointment and lotion, or a combination of these forms, to the desired treating area on the skin, then treating the skin area with drug using the said microdevice.

In yet a further embodiment, one can pre-coat a microneedle shaft with a composition that contains active substances. The coated microneedles are applied to the skin and stay on the skin for the entire period of treatment. The rate of through skin transport can be measured using in vitro or in vivo methods known in the art.

### Applicator

In some embodiments, an area of skin can be pre-treated by the microdevice described herein using an applicator. An "applicator device" or "applicator" as used herein refers to a device or object used to generate nanoconduits in the skin. The applicator preferably comprises a driving structure, element, mechanism, and/or means for generating nanoconduits in the skin. The applicator is preferably used in conjunction with a surface, patch, or reservoir comprising an agent to be delivered. The applicator helps to both deliver agent and generate nanoconduits at a single predetermined skin area at a time. Accordingly, in an alternative embodiment, the applicator may simultaneously generate nanoconduits in the skin area and apply a pre-applied agent.

The driving mechanism may be spring based, solenoid based, electromechanical based, magnetic based, artificial muscle based or based on any other mechanism useful to cause a microdevice along a path to penetrate the stratum corneum. In one embodiment, the applicator may comprise a housing, a movable holder configured to hold a
microdevice, a driving mechanism, and a depth stop.

In another embodiment, the applicator device comprises an element that generates the plurality of nanoconduits in the stratum corneum by sonoporation, electroporation, laser light, microdevice, or combinations thereof.

In one embodiment, the spring powered applicator is particularly attractive because it provides a driving force needed to insert microneedles, or slice skin surface using microknives and microblades without relying on any independent power source. The use of such a device calls upon the user to load a disposable element with the microdevice attached to one end into the applicator. The applicator can launch the microneedle into skin or slice through skin surface quickly to create reproducible nanoconduits. These nanoconduits are created by displacing the tissue during the application process. Such an applicator device may be coupled with a means for applying the active agent either before, during, or after the nanoconduits are generated.

According to the invention, the applicator employs vibration of one or more frequencies to create an impact force to generate the nanoconduits. For example, in one embodiment, the applicator may contain an exchangeable head, connecting to a battery- powered motor with two eccentric wheels, transforming rotation to vibration. The vibration can create an impact to the skin through a plurality of microneedles mounted on top of the exchangeable head. The frequency of vibration depends on rotation speed and mass distribution of the eccentric wheels and can be in the range of about 10 Hz to about 50000 Hz. In some embodiments, the frequency range can be between about 1000 and about 8000 Hz. An ordinary artisan can design and make an applicator accordingly. Some examples of designing an applicator for various uses are described in U.S. Patent Nos. 390,089; 1,512,981; 1,657,312; 1,683,851; 1,780,757; 1,790,962; 1,900,609; 2,411,196;; 4,237,911; 4,979,525; 5,054,149; 5,095,924; 5,215,193; 5,328,682; 5,713,492;
5,738,122; D416,387; 6,092,252; and 6,220,253.

In some embodiments, the applicator for mechanical skin treatment comprises:
(a) a housing having a plurality of walls defining an interior space, the interior space having an upper opening permitting selective access to the interior space of the housing, a cover member being removably couplable to the housing such that the cover is for closing the upper opening of the interior space of the housing; and
(b) a plurality of microneedles head portion connected to a base portion being removably insertable into the interior space of the housing, each of the applicators being adapted for aiding a user to treat skin;
   wherein the plurality of the applicator including a microneedle array assembly comprises:
   (i) the microneedle array assembly being adapted for selectively treating skin, and
   (ii) the microneedle assembly having a head portion and a base portion, the head portion being selectively couplable to the base portion such that the base portion is insertable into the interior space of the housing, the base portion of the microneedle array assembly having a pair of depressions, each of the depressions extending along a portion of a length of the base portion, one of the depressions being positioned opposite the other of the depressions such that the depressions are adapted for receiving finger tips of a hand of the user for inhibiting slipping of the base portion from the hand of the user of the applicator.

According to the invention, the base portion of the applicator described above further comprises a motor assembly being positioned in the base portion, the head portion having a drive assembly being positioned in the head portion, the drive assembly being
operationally coupled to a base portion, the base portion outwardly extending from an upper end of the head portion, the motor assembly being operationally coupled to the drive assembly such that the motor assembly is for actuating the drive assembly, the drive assembly being for oscillating the base portion when the drive assembly is actuated by the motor assembly.

In some embodiments, the applicator described above can further comprise a head portion having a plurality of microneedles extending from the base portion, the microneedles being adapted for treating the skin when the microneedle head portion is oscillated by the drive assembly.

In some embodiments, the applicator described above can further comprise a motor assembly having a motor, the motor having a shaft extending from the motor, the motor being for actuating the shaft, the shaft being for operationally coupling to the drive assembly of the base and head portions such that actuation of the shaft actuates the drive assembly, a power source being operationally coupled to the motor such that the power supply is for providing power to the motor.

According to the invention, the applicator described above includes a heavy eccentric mass designed to produce vibration upon actuation of the motor, wherein the motor is actuated to bring the base and head portions into vibration so that skin treatment is practiced through the aid of the vibration, In this aspect, the microneedle application method comprises:
predetermining respective weights of the electric applicator and the heavy eccentric mass as well as an eccentric location of the center of gravity of the heavy eccentric mass; establishing an output of the motor at about 1000-15000 rpm in accordance with the predetermined conditions; producing a vibration of about 1000-15000 rpm by actuating the motor;
conducting the vibration to tips of microneedles on the head portion to increase a pressing force acting along an axial direction of the base and head portion by the use of a minute circular ring connecting to the handle part and pressing against a skin area needing treatment.

In some embodiments, the applicator described above can further include a motor assembly having a switch, the switch being operationally coupled between the power supply and the motor, the switch being for controlling power from the power supply to the motor.

In another aspect that is not part of the claimed invention, the present subject matter relates to a kit for delivering an agent to a mammal, comprising
(a) a microdevice comprising a structure selected from microneedles, microblades, microknives, and combinations thereof;
(b) a wet formulation comprising a bioactive agent; and
(c) a mechanism to provide for a driving force sufficient to form nanoconduits on the skin, lined with tissue displaced by the driving force.

In this regard, the driving force can be, but is not limited to, ultrasound, iontophoresis, radio frequency, laser light, heat gradient, or a combination thereof.

In addition, the present kits may further comprises an applicator of the microdevice for applying the microdevice to an area of skin of a mammal; and/or a driving force mechanism for driving the bioactive agent to transport through the stratum corneum of the area of skin into the mammal. The driving force mechanism can comprise ultrasound, radio frequency, heat gradient, laser light, iontophoresis device, or a combination thereof. In a preferred embodiment, the applicator is a mechanical applicator.

The present kits are particularly useful in treating a mammal having a medical
condition. Some potential medical conditions treatable by the present kits include, but are not limited to, chronic back pain, a cancer, pre-surgery pain management, operation room pain management, cancer pain, post-surgery pain and lower back pain, and post-surgery pain management.

In this regard, the agent used in the kits can be, but is not limited to, a natural or synthetic vaccine selected from the group consisting of proteins, peptides, paclitaxel, docetaxel, vaccines, protein vaccines, peptide vaccines, gene vaccines, DNA vaccines, and combinations thereof. Further, the vaccine can be against influenza (flu), diphtheria, tetanus, pertussis (DTaP), measles, mumps, rubella (MMR), hepatitis B, polio, haemophilus influenzae type b, chickenpox, tuberculosis, anthrax, yellow fever, rabies, AIDS, cancers, meningococcus, SARS, and/or cholera, hi the alternative, the agent is a pain relieving agent. In this regard, the pain relieving agent can be lidocaine, tetracaine, dyclonine, or a combination of thereof.

The formulation used in the present kits can comprise elastic liposomes encapsulating the agent. The elastic liposomes can optionally comprise deformable nanoparticles. In the alternative, the formulation in the present kits does not comprises elastic liposomes. In addition, the formulation can be a topical or systemic delivery formulation selected from a skin patch, cream, ointment, or lotion. In a preferred embodiment, the formulation is a wet skin patch.

### Active Agents

In one aspect, active agents or active substances that can be delivered using microdevices are therapeutic agents. The term "therapeutic agent" is used here to refer to an active agent that can treat, prevent, and ameliorate a body condition or skin condition that needs treatment. A list of examples includes: drugs, natural or synthetic vaccines, peptides, proteins, genes, DNAs, RNAs, nutraceuticals and cosmetics. The drugs can be
administered topically or systemically. Examples of the drugs useful herein as active agents include, but are not limited to, antibiotics, hormones, steroids, anti-inflammatory drugs, protein drugs, anesthetics, protein vaccines, peptide vaccines, gene vaccines, DNA vaccines, paclitaxel, docetaxel, insulin, DNA drugs whether natural or synthesized, such as Recombinant Erythropoietin (rhEPO), Taxol (R), Interferon-alpha, Interferon-alpha-lb, Interferon beta, Interferon gamma, Emla(R), Fluorouracil, Lidocaine, Salicylic acid, Pureriran, eflornithine hydrochloride, spironolactone, flutamide, insulin, carnitine, nanoparticle drugs, Epidural, recombinant human parathyroid hormone, growth hormone, calcitonin, Follicle Stimulation Hormone, thyroid, Cortisol, estrogen, progesterone, testosterone, and combinations thereof. Non-limiting examples of vaccines active agents include, but not limited to: vaccine against influenza (flu), diphtheria, tetanus, pertussis (DTaP), measles, mumps, rubella (MMR), hepatitis B, polio, haemophilus influenzae type b, chickenpox, tuberculosis, anthrax, yellow fever, rabies, AIDS, cancers, meningococcus, SARS, cholera, and combinations thereof. More examples of cosmetic substances as active agents include, but not limited to: botulinum toxin type A, hyaluronic acid and its derivatives, acetyl hexapeptide-3, vitamin A, vitamin C, vitamin E, alpha-hydroxyacids, collagen, hormones, and combinations thereof. Diagnostic reagents are also included and contemplated herein. Examples include, but are not limited to, quantum dots, functionalized nanoparticles, magnetic particles, and combinations thereof.

The dosage of the agent can vary according to the medical condition(s) being treated. The effective amount of an agent that has been well established in the art can be publicly available. Such information can be obtained from the U.S. Food and Drug Administration (FDA), e.g., FDA website. For example, LidoDerm(R) info can be found in this link: http://www.fda.gov/medwaTCH/SAFETY/2006/Apr PIs/Lidoderm PI.[rho]df#search=%221i doderm%20dosage%22.

In some embodiments, the agent is a pain relieving drug for neuropathic or nociceptive pain management. Such pain relieving drug includes, but are not limited to, Lidocaine; Prilocaine, dyclonine, Tetracaine, Ibuprofen; Acetaminophen; Capsaicin; EMLA(R); Tramadol (Ultram); Gabapentin, Tramadol hydrochloride, Corticosteroids, Sufentanil, Clonidine, Bupivacaine, Tricyclic antidepressants, opioid analgesics such as morphine, Hydromorphone, naloxone (Narcan), Talwin, Nubain, Stadol, Fentanyl, Meperidine, Hydrocodone, Codeine, Oxycodone; non-selective NSAIDs such as Celecoxib (Celebrex), rofecoxib (Vioxx), valdecoxib (Bextra); and combinations thereof. In some embodiments, the pain relieving drug described herein can specifically include or exclude any of the drug/agents listed herein.

In some embodiment, the active agent can be muscle relaxants, which include, but are but not limited to, Benzodiazepines; Methocarbamol; Carisoprodol; Chlorzoxazone; Metaxalone; Cyclobenzaprine, and combinations thereof. In some embodiments, the muscle relaxants described herein can specifically include or exclude any of the drug/agents listed herein.

### Drug delivery

In one aspect, the present subject matter provides a device for delivery of a therapeutic active agent as defined above across the skin barrier, i.e., the stratum corneum layer. Once the substances pass the stratum corneum, there is less resistance for the substances to diffuse into the subsequent layers of the skin: i.e., the epidermis and dermis. The substances will be absorbed by micro blood vessels and lymphatics in the dermis layer and delivered to the entire human body. Microdevices disclosed herein can enhance delivery through skin penetration of molecules of molecular weight lower than about 500 Daltons. In some embodiments, the present microdevices can also enable delivery through skin transport of large molecules of a molecular weight higher than about 500 Daltons. The molecular weight of Bovine Serum Albumin is about 66,000 Dalton. The molecular weight of Botulinum Toxin Type A is about 150,000 Dalton and the molecular weight of Interferon-Alpha- Ib is about 17,000 Dalton.

In this regard, the present systems permit the agent to permeate the skin at least 2 times faster, at least five times faster, or at least 10 times faster than in a system where the formulation is applied to an area of skin which does not contain any generated nanoconduits. hi this regard, the agent can permeate the skin to cumulative amounts at least two times higher, at least five times higher, or at least 10 times higher than in a system where the formulation is applied to an area of skin which does not contain any generated nanoconduits.

In some embodiments, the drug delivery of the present subject matter can be achieved by preparing an area of skin to generate a prepared area of skin and then applying an agent or drug to the prepared area of skin to allow a pre-defined amount of the drug or agent to pass through the stratum corneum of the prepared area of skin.

In some embodiments, the prepared area of skin can be prepared using a device, e.g., a spring-powered mechanical applicator to apply microneedles to an area of skin. The mechanical applicator can be any structure or design and can cause a mechanical force to be applied to the microneedle against the area of skin to generate pores or channels in the stratum corneum in the area of skin in a pre-defined size and depth. The size and depth of the pores or channels can facilitate the release of controlled amount of an agent or drug through skin, hi some embodiments, the controlled amount of agent is contained in a patch that is applied to the targeted area of skin comprising nanoconduits.

In some embodiments, the prepared area of skin can be further treated using an ultrasound device or a mechanical vibrator to apply microneedles to an area of skin. The ultrasound device or mechanical vibrator can cause a pre-set mechanical force to be applied to the microneedle against the area of skin to generate pores or channels in the stratum corneum in the area of skin in a pre-determined size and depth. The size and depth of the pores or channels can provide for controlling the amount of an agent or drug of delivery. It is noteworthy that the ultrasound device or mechanical vibrator can be an effective way to perforate an elastic skin tissue to generate pores or channels in a predefined size and/or depth.

In some embodiments, the prepared area of skin can be prepared in a pre-defined size or dimension (e.g., a dimension of 1 cm x 1 cm) using an array of microknives or microblades by slicing or lacerating the stratum corneum in an area of skin to generate nanochannels in a pre-defined depth and/or dimension. The dimension and/or depth of the laceration and the dimension of the prepared area of skin can provide for controlling the amount of an agent or drug.

Allowing an agent or drug to pass through the stratum corneum of a prepared area of skin can be achieved by a variety of mechanisms. For example, the allowing can be achieved by diffusion of the agent or drug from a topical composition (e.g., a formulation such as lotion, cream, gel patch, ointment or skin patch) into the body of a patient or user via the prepared area of skin. In some embodiments, the agent may permeate into the skin and past the stratum corneum by (a) diffusing from the formulation and into the nanoconduits, (b) driving the agent into the nanoconduits by applying a driving force, or (c) a combination thereof. The nanoconduits are generated in the skin by a microdevice or element applied to the skin with sufficient driving force, wherein the sufficient driving force is attained by manual force or mechanized force. Manual force refers to moving the
microdevice by hand and/or by hand with the assistance of a lever or lever action. Mechanized force refers to moving the microdevice or element by an energy-assisted means. An energy-assisted means for achieving a driving force may include, for example, using a spring-powered mechanical device, a motor-powered mechanical device, a mechanical or ultrasonic vibrating device, a gas pressure or liquid pressure generating device, or combinations thereof. In this regard, the driving force can be mechanical, electromechanical, iontophoresis, sonophoresis, radiofrequency (RF), laser light, heat gradient, or a combination thereof to actively drive agents through the skin.

Iontophoresis, sonophoresis, radiofrequency (RF) or heat are well developed mechanisms for promoting or enhancing drug delivery. Some examples of iontophoresis systems in drug delivery are described in http://www.vvteris.com and http://www.iomed.com. Some examples of sonophoresis systems in drug delivery are described in www.sontra.com (Becker B, Helfrich S, Baker E, et al. Ultrasound with topical anesthetic rapidly decreases pain of intravenous cannulation. Academic Emergency Medicine 2005; 12:289-295; Katz N, Shapiro D, Herrmann T, et al., Rapid onset of cutaneous anesthesia with EMLA cream after pretreatment with a new ultrasound-emitting device. Pain Trials Center, Brigham and Women's Hospital, Boston, Massachusetts; Mitragotri S, Kost J, Low frequency sonophoresis: A Review. Advanced Drug Delivery Reviews 2004; 56:589-601. Some examples of RF systems in drug delivery are described in http^/www.transphanna-medical.com/references.html. Some examples of drug delivery systems using heat are described in http://www.zars.com.

### Topical or systemic delivery of cosmetic substances

It is known to one in the art that certain substances have specific functions as cosmetics. For example, Botulinum Toxin Type A is a toxin that blocks neuromuscular
transmission when it is injected in small amounts (e.g., 10 units per 0.1 ml injection volume) into specific muscles to treat and reduce wrinkles on the face. The maximum dosage recommended as a single injection for any one muscle at any spot is 25 units. If overdosed or the injection is incorrectly performed, the patient can be left with an immobile face or droopy eyelids till the effect of the injection wears off. The side effects include numbness, swelling and headaches. Administered through the present microdevices, it is possible to provide a controlled release of Botulinum Toxin Type A and keep an optimal local concentration to achieve the best result while minimizing the side effects. In a preferred embodiment, a gel patch with botulinum toxin type A is applied to the skin pre-treated with a microneedle array. No through skin transport was observed without application of microdevices while significant through skin transport of botulinum toxin type A was observed using the said microdevice.

Transdermal delivery of an agent through skin treated by the microdevice described herein has less dependency on molecular weight of the agent. Using the methods described herein, practically any cosmetic substances can be delivered using microdevices herein. Local concentrations can be adjusted through loading and compositions for controlled release, as well as a combination of microneedle height, density, size and shape. In one embodiment, one can deliver hyaluronic acid gel through diffusion enhanced by nanoconduits generated by microdevices. Hyaluronic acid is a substance that exists naturally in the body. A major important function of hyaluronic acid is to carry and bind water molecules. Stabilized non-animal hyaluronic acid does not contain animal protein and does not require a skin test prior to treatment. It is thus a preferred embodiment herein to use microdevices to deliver locally stabilized non-animal hyaluronic acid to treat wrinkles and facial lines.

Yet, in a further embodiment of the present subject matter, one can locally deliver
collagen by microneedles, e.g., for allergic skin test and controlled release of collagen into the skin.

Yet another embodiment of the present subject matter is to provide for local delivery of acetyl hexapeptide-3. This molecule is a non-toxic, non-irritant compound that modulates the excessive stimulation of the facial muscles, relaxing facial tension and it can reduce and prevent the formation of new wrinkles due to over-stimulation of facial muscles. More examples of cosmetic agents useful herein include but are not limited to: vitamin A, vitamin C, vitamin E, alpha-hydroxyacids, hormones, and combinations thereof.

### Delivery of vaccines

The microdevice provided herein can be used for topical or systemic delivery of vaccines below the stratum corneum layer, but such use is not part of the claimed invention. The type of vaccines includes conventional vaccines as well as protein, peptide, DNA vaccines and the like as previously described. Vaccination can be performed by treating a skin site with the microdevice and then delivering a vaccine composition to a user.

### Delivery of large molecules

In some embodiments, the microdevice provided herein can be used for topical or systemic delivery of large molecule drugs. The drug can be a protein or peptide. In some embodiments, the drug can be a chemical drug with a relatively high molecular weight. As used herein, the term large molecule refers to a drug having a molecular weight higher than about 300 Daltons. For example, the molecule can have a molecular weight higher than about 500 Daltons, higher than about 1000 Daltons, higher than about 5,000 Daltons, higher than about 10,000 Daltons, higher than about 20,000 Daltons, higher than about 50,000 Daltons, higher than about 100,000 Daltons, higher than about 200,000 Daltons, higher than about 500,000 Daltons, or higher than about 1 ,000,000 Daltons.

### Pain management

The microdevice described herein can be used for pain management, but such use is not part of the claimed invention. The microdevice can be used to facilitate transdermal delivery of a pain relieving agent or a combination of them so as to treat, reduce or prevent pain. In some embodiments, a skin site can be treated with the microdevice and then a pain relieving agent or drug composition can be applied to the treated site, allowing transdermal delivery of these agents to a user. The pain relieving agent can be any pain relieving agent approved by FDA or used in medical practice elsewhere in the world. In some embodiments, the pain relieving drug can be, but are not limited to, NSAIDs, COX-2 inhibitors, steroids, muscle relaxants.

The pain management can be carried out according to a management regime prescribed by a treating doctor. For example, in some embodiments, the pain management is chronic or acute pain management. The pain management regime can be but is not limited to, lower back pain, chronic back pain, post-herpetic neuralgia, cancer pain, diabetic neuropathy, phantom limb pain, spinal stenosis/sciatica, spinal mets, HIV pain, post surgery pain, pre-surgery preparation, operation room pain management, and pain caused invasive medical procedures such as needle injection or cannulation. Chronic back pain is particularly preferred in this regard.

Different from prior art, the current subject matter involves the topical or systemic delivery of a pain relief agent to deep tissues through assistance of a combination of active transdermal delivery methods such as sonophoresis, iontophoresis, laser ablation, radio frequency or heat treatment after the stratum corneum is treated with the present microdevices.

In another aspect, the present systems and methods are useful for treating cancer.

### Controlled Release

The microdevices herein preferably deliver drug molecules through skin at a rate that is sufficient to maintain a therapeutically useful concentration in plasma. The size, density, shape and length of the microdevices can be adjusted to meet the delivery requirements. The microdevices can be further coated with a composition that contains active therapeutic molecules, or vaccines, or cosmetic substances, together with polymer binders such as chitosan, carbopol 934P, cellulose and starch to form a dry film. Additional additives of binders, rheology modifiers, surface active agents, stabilizer, rehydration agents may be used. The special composition can control the dissolve rate of the active drug molecule and regulate the drug release rate. In some embodiments, the microdevices may be integrated with embedded microfluidic channels that connect to microreservoirs. In other embodiments, the microdevices do not comprise embedded microfluidic channels or microreservoirs.

In this regard, the present the methods result in a plateau plasma concentration of agent, wherein the plateau is preferably maintained within 80% of the peak concentration from 10 hours after first contact and up to at least 24 hours after first contact. The plateau can be maintained within 80% of the peak concentration from 10 hours after first contact and up to at least 48 hours after first contact.

Accordingly, the agents herein have a "drug permeation", meaning as the amount of agent (mg, or mmol) diffused across the skin barrier within a defined time.

### The Integrated Sensors

It is another aspect of the present subject matter that does not belong to the claimed invention to optionally provide a device in which clinical biosensor and/or sensor arrays are fabricated in close vicinity of the HARMS structures. For example, microneedles can collect an extremely low sample volume of body fluids from a patient and allow rapid point-of-care analysis of body fluids.

In one embodiment, the sample volume extracted is below 0.1 microliter, typically around 0.01 microliter.

### Methods for HARMS Fabrication

The HARMS may be fabricated using MEMS (Micro-Electro-Mechanical Systems) microfabrication technology. The typical fabrication process involves lithography, wet etch and dry etch, thin film deposition and growth, electroplating, as well as injection molding and hot embossing. One example of a fabrication method useful herein is to use the Bosch process that allows deep Si etch (www.oxfordplasma.de/process/sibo 1.htm).

This process forms HARMS suitable either as a device body or as a mold for further processing. The aspect ratio is preferably higher than 5:1, independent of feature size and pattern shape as long as the features can be defined by lithography. Another fabrication method is KOH or TMAH wet etch of single crystal Si substrate that is <100> orientation or <110> orientation. Yet another fabrication method useful herein is using HF solution to electrochemically form porous Si structures (www.techfak.uni-kiel.de/matwis/amat/poren/ps .html).

Metals can be used for the fabrication of HARMS through a maskless process called electropolishing starting from a structure fabricated by traditional machining methods such as cutting, electro-discharge machining, milling, grinding, polishing and drilling (www.najet.com and www.fischion.com/product support/model 110 application notes.asp).

Use of any single method herein or a combination of these methods as further disclosed in the examples below led to the form of desired HARMS disclosed in the current subject matter.

### EXAMPLES

### Example 1. Delivery of docetaxel with combination of microneedle with flexible liposome nanoparticles (not according to the invention).

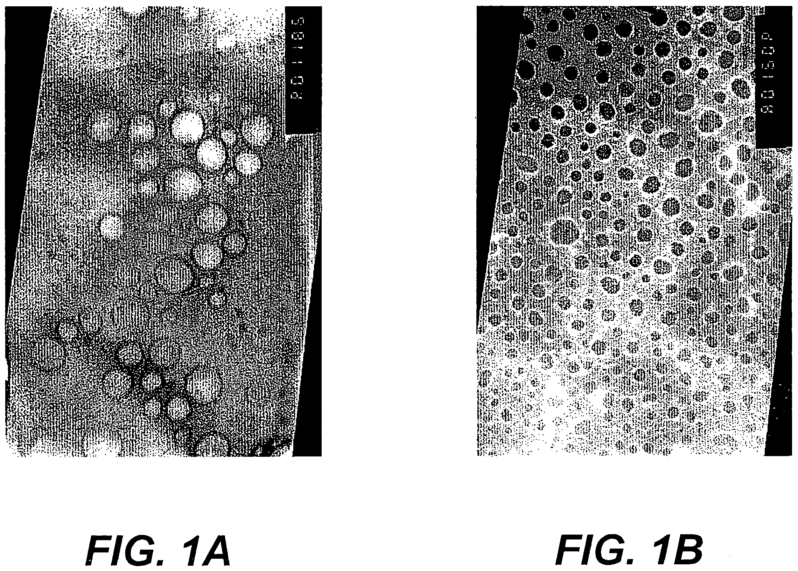

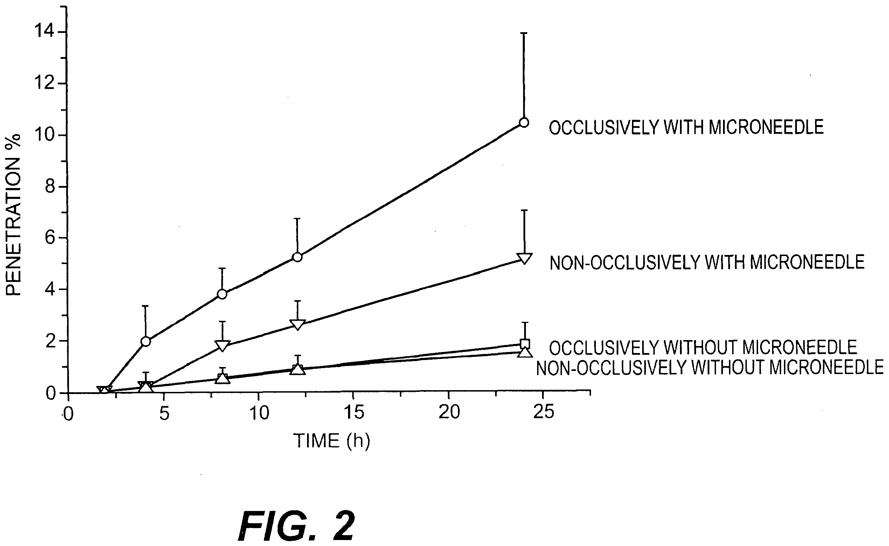

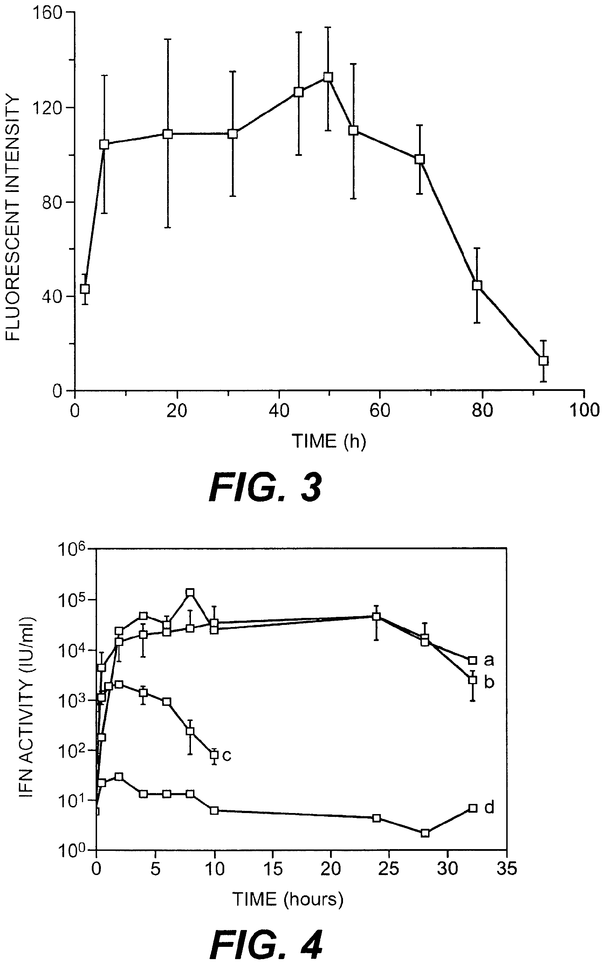

Figures 2 and 3 showed the efficacy of transdermal delivery of agents of the present subject matter. In the test shown in Figures 2 and 3, an area of skin was pre- treated with the microneedles described above. Then a formulation of a fluorescence labeled albumin (molecular weight is 66,000) was applied and successfully transport them through skin (Figure 3). The pore formed by the microneedles will not completely be closed within 72 hours after application of the microneedles. Figure 2 shows Penetration (%) of docetaxel in elastic liposomes with or without microneedle.

### Example 2. Delivery of interferon with combination of microneedle (not according to the invention).

Figure 4 shows delivery of interferon via different methods. The effectiveness of various delivery methods was assessed by measurement of interferon activity: (a) microneedle with a wet interferon gel on the microneedles, (b) microneedle with a wet interferon gel patch on skin pre-treated with microneedles, (c) subcutaneous injection, and (d) wet interferon gel without microneedle as control sample.

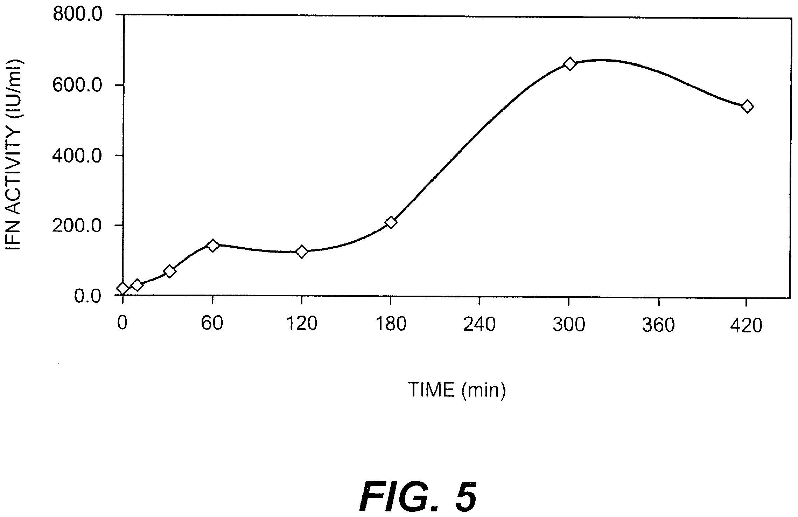

Figure 5 shows delivery of interferon with a dry formulation. As Figure 5 shows, when the patch is dried, the delivery rate dropped dramatically.

In sum, Figures 4 and 5 show that delivery of interferon using a dry patch is less effective as it is using a wet patch.

### Example 3. Measuring permeation across skin.

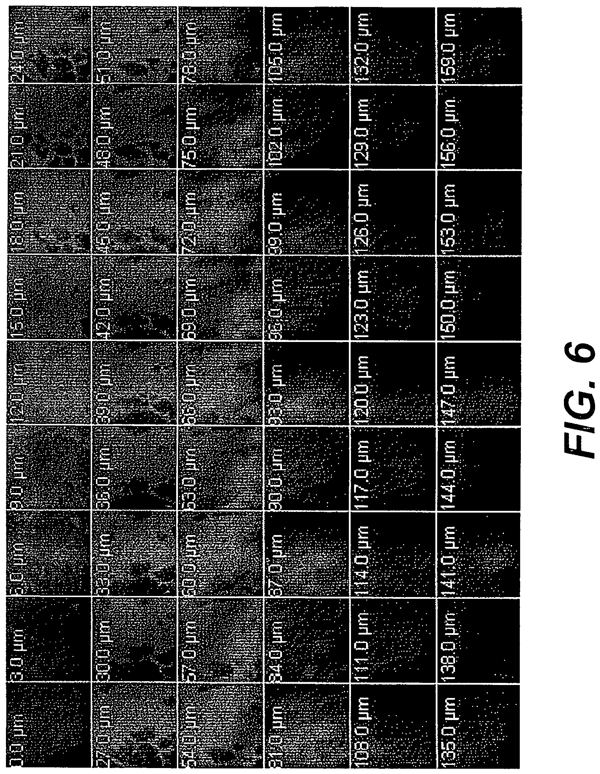

The confocal laser photomicrographs of Fig. 6 were obtained by applying microneedles array to the skin using an applicator, then allow nanoparticles containing Rhodamine B, which emits red fluorescence, to diffuse through the skin for less than 30
min. These pictures are sections of skin at various depths. The depths are marked on top of each picture. From these pictures, it's clearly that the high concentration of the fluorescence probe can be found between 10-100 um deep from the skin surface.

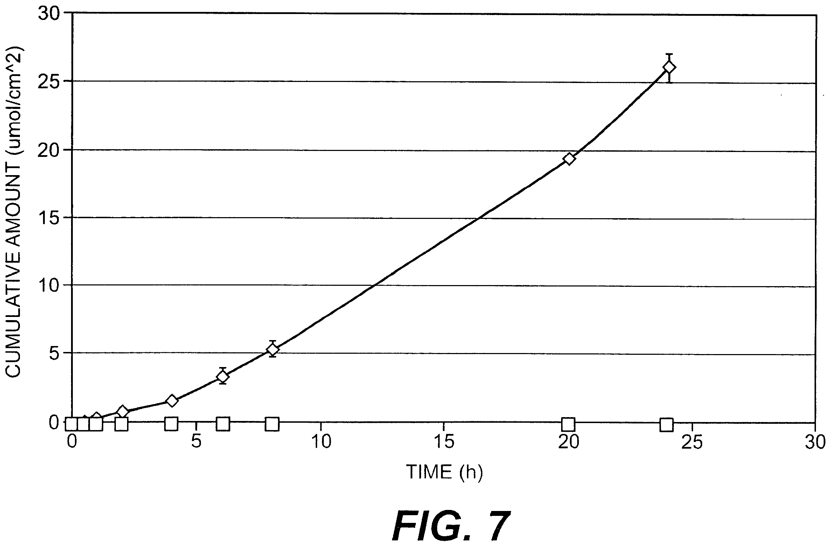

Another example of permeation measurement is shown in Fig. 7. A fluorescently- labeled insulin was in one supply reservoir and the receiving reservoir has no insulin. The two reservoirs were separated by skin barrier pretreated with nanoconduits generated by microneedles. The fluorescence intensity on the receiving reservoir side was directly proportional to the amount of insulin that permeated the skin.

While particular embodiments of the present subject matter have been shown and described, it will be obvious to those skilled in the art that changes and modifications can be made without departing from this subject matter in its broader aspects.

## Claims

1. An applicator device for mechanical skin treatment comprising:
a housing having a plurality of walls defining an interior space, the interior space having an upper opening permitting selective access to the interior space of the housing, a cover member being removably couplable to the housing such that the cover is for closing the upper opening of the interior space of the housing; and
a plurality of microneedles head portion connected to a base portion being removably insertable into the interior space of the housing, each of the applicators being adapted for aiding a user to treat skin;
wherein the plurality of the applicator including a microneedle array assembly comprises:
the microneedle array assembly being adapted for selectively treating skin, and the microneedle assembly having a head portion and a base portion, the head portion being selectively couplable to the base portion such that the base portion is insertable into the interior space of the housing, **characterized in that** the base portion of the microneedle array assembly has a pair of depressions, each of the depressions extending along a portion of a length of the base portion, one of the depressions being positioned opposite the other of the depressions such that the depressions are adapted for receiving finger tips of a hand of the user for inhibiting slipping of the base portion from the hand of the user of the applicator;
**in that** the base portion of the microneedle applicator further comprises a motor assembly being positioned in the base portion, the head portion having a drive assembly being positioned in the head portion, the drive assembly being operationally coupled to a base portion, the base portion outwardly extending from an upper end of the head portion, the motor assembly being operationally coupled to the drive assembly such that the motor assembly is for actuating the drive assembly, the drive assembly being for oscillating the base portion when the drive assembly is actuated by the motor assembly and
**in that** the applicator comprises a heavy eccentric mass designed to produce vibration upon actuation of the motor, wherein the motor is actuated to bring the base and head portions into vibration so that skin treatment is practiced through the aid of the vibration.

2. Use of an applicator device according to claim 1 for delivering at least one cosmetic agent to a mammal in need thereof, comprising
applying the applicator device to an area of skin on the mammal to cause the device to generate a plurality of nanoconduits in stratum corneum of the area of skin, thereby creating a prepared area of skin;
applying a wet formulation to the prepared area of skin, wherein the wet formulation comprises at least one agent, wherein the agent is separate from the microdevice; and
allowing an effective amount of the at least one agent to enter the mammal via the nanoconduits in the stratum corneum;
wherein the applicator device comprises an element capable of generating a plurality of nanoconduits in the stratum corneum.

3. The use of claim 2, wherein the agent is applied as a wet formulation while in the form of a lotion, cream, gel patch, skin patch, ointment, elastic liposome, or a combination thereof.

## Patentansprüche

1. Applikatorvorrichtung zur mechanischen Hautbehandlung, umfassend:
ein Gehäuse mit mehreren Wänden, die einen Innenraum definieren, wobei der Innenraum eine obere Öffnung hat, die selektiven Zugang zum Innenraum des Gehäuses zulässt, ein Abdeckelement, das abnehmbar mit dem Gehäuse gekoppelt werden kann, so dass die Abdeckung zum Schließen der oberen Öffnung des Innenraums des Gehäuses ist; und
einen Kopfteil mit mehreren Mikronadeln, der mit einem Basisteil verbunden ist, der abnehmbar in den Innenraum des Gehäuses einsetzbar ist, wobei jeder der Applikatoren zum Unterstützen eines Benutzers bei einer Hautbehandlung ausgeführt ist;
wobei die mehreren Applikatoren mit einer Mikronadelanordnungsbaugruppe Folgendes aufweisen:
die Mikronadelanordnungsbaugruppe, die zum selektiven Behandeln von Haut ausgeführt ist, und die Mikronadelbaugruppe, die einen Kopfteil und einen Basisteil hat, wobei der Kopfteil selektiv mit dem Basisteil gekoppelt werden kann, so dass der Basisteil in den Innenraum des Gehäuses einsetzbar ist,
**dadurch gekennzeichnet, dass** der Basisteil der Mikrowellenanordnungsbaugruppe ein paar Vertiefungen hat, wobei jede der Vertiefungen sich an einem Teil einer Länge des Basisteils entlang erstreckt, wobei eine der Vertiefungen gegenüber der anderen der Vertiefungen positioniert ist, so dass die Vertiefungen zum Aufnehmen von Fingerspitzen einer Hand des Benutzers ausgeführt sind, um ein Herausrutschen des Basisteils aus der Hand des Benutzers des Applikators zu verhindern;
dadurch, dass der Basisteil des Mikronadelapplikators ferner eine Motorbaugruppe aufweist, die in dem Basisteil positioniert ist, der Kopfteil eine Antriebsbaugruppe hat, die im Kopfteil positioniert ist, die Antriebsbaugruppe funktionell mit einem Basisteil gekoppelt ist, der Basisteil sich von einem oberen Ende des Kopfteils nach außen erstreckt, die Motorbaugruppe funktionell mit der Antriebsbaugruppe gekoppelt ist, so dass die Motorbaugruppe zum Betätigen der Antriebsbaugruppe dient, die Antriebsbaugruppe zum Schwingenlassen des Basisteils dient, wenn die Antriebsbaugruppe von der Motorbaugruppe betätigt wird, und
dadurch, dass der Applikator eine schwere exzentrische Masse aufweist, die dafür ausgelegt ist, bei Betätigung des Motors Schwingungen zu erzeugen, wobei der Motor betätigt wird, um die Basis- und Kopfteile in Schwingung zu versetzen, so dass die Hautbehandlung mithilfe der Schwingung praktiziert wird.

2. Verwendung einer Applikatorvorrichtung nach Anspruch 1 zur Abgabe wenigstens eines kosmetischen Wirkstoffs an ein diesen benötigendes Säugetier, umfassend:
Anwenden der Applikatorvorrichtung auf eine Hautfläche an dem Säugetier, um die Vorrichtung zum Erzeugen mehrerer Nanoleitungen in der Hornschicht der Hautfläche zu veranlassen, wodurch eine vorbereitete Hautfläche geschaffen wird;
Auftragen eines nassen Präparats auf die vorbereitete Hautfläche, wobei das nasse Präparat wenigstens einen Wirkstoff beinhaltet, wobei der Wirkstoff von der Mikrovorrichtung separat ist; und
Eindringenlassen einer wirksamen Menge des wenigstens einen Wirkstoffs in das Säugetiere über die Nanoleitungen in der Hornschicht;
wobei die Applikatorvorrichtung einElement aufweist, das mehrere Nanoleitungen in der Hornschicht erzeugen kann.

3. Verwendung nach Anspruch 2, wobei der Wirkstoff als ein nasses Präparat aufgetragen wird, während es die Form einer Lotion, einer Creme, eines Gel-Pflasters, eines Haut-Patchs, einer Salbe, von elastischem Liposom oder einer Kombination davon hat.

## Revendications

1. Dispositif applicateur destiné au traitement mécanique de la peau comprenant :
- un boîtier ayant un ensemble de parois définissant un volume interne, ce volume interne ayant une ouverture supérieure permettant son accès sélectif, un élément d'obturation pouvant être couplé de façon amovible au boîtier de sorte qu'il permette de fermer l'ouverture supérieure du volume interne, et
- un ensemble de parties de tête à micro-aiguilles connecté à une partie de base et pouvant être inséré de façon amovible dans le volume interne du boîtier, chacun des applicateurs étant susceptible d'aider un utilisateur à effectuer un traitement de la peau,
- l'ensemble d'applicateurs comportant un ensemble de réseaux de micro-aiguilles dans lequel :
- l'ensemble de réseaux de micro-aiguilles est susceptible d'effectuer un traitement sélectif de la peau et l'ensemble de micro-aiguilles a une partie de tête et une partie de base, la partie de tête pouvant être sélectivement couplée à la partie de base de sorte que la partie de base puisse être insérée dans le volume interne du boîtier, **caractérisé en ce que**
- la partie de base de l'ensemble à réseaux de micro-aiguilles a une paire de cavités, chacune de ces cavités s'étendant le long d'une partie de la longueur de la partie de base, l'une de ces cavités étant située à l'opposé de l'autre cavité de sorte que les cavités soient adaptées pour recevoir le bout des doigts de la main de l'utilisateur pour empêcher le glissement de la partie de base de la main de l'utilisateur de l'applicateur,
- la partie de base de l'applicateur à micro-aiguilles comprend en outre un ensemble moteur monté dans la partie de base, la partie de tête ayant un ensemble d'entraînement monté dans la partie de tête, l'ensemble d'entraînement étant opérationnellement couplé à la partie de base, la partie de base s'étendant vers l'extérieur à partir de l'extrémité supérieure de la partie de tête, l'ensemble moteur étant couplé opérativement à l'ensemble d'entraînement de sorte que l'ensemble moteur soit susceptible d'actionner l'ensemble d'entraînement, l'ensemble d'entraînement étant destiné à faire osciller la partie de base lorsque l'ensemble d'entraînement est actionné par l'ensemble moteur, et
- l'applicateur comprend une masse excentrique lourde susceptible de produire des vibrations suite à l'actionnement du moteur, le moteur étant actionné pour faire vibrer la partie de base et la partie de tête de sorte que le traitement de la peau puisse être effectué à l'aide des vibrations.

2. Utilisation d'un dispositif applicateur conforme à la revendication 1, pour distribuer au moins un agent cosmétique à un mammifère qui en a besoin, comprenant des étapes consistant à :
- appliquer le dispositif applicateur sur une zone de la peau du mammifère de sorte qu'il crée un ensemble de nano-conduites dans le stratum corneum de la zone de la peau, de façon à obtenir une zone de peau préparée,
- appliquer une formulation humide à la zone de peau préparée, la formulation humide comprenant au moins un agent, cet agent étant séparé du micro-dispositif,
- faire entrer une quantité efficace de l'agent dans le mammifère via les nano-conduites situées dans le stratum corneum, le dispositif applicateur comprenant un élément susceptible de créer un ensemble de nano-conduites dans le stratum corneum.

3. Utilisation conforme à la revendication 2, selon laquelle l'agent est appliqué sous la forme d'une formulation humide constituée par une lotion, une crème, un patch sous forme de gel, un patch cutané, une pommade, un liposome élastique ou une combinaison de ces éléments.
